# EUROPEAN PATENT SPECIFICATION

(11) **EP 1 820 794 B1**
(45) Date of publication and mention of the grant of the patent: **05.01.2011**
(21) Application number: 05778843.2
(22) Date of filing: 14.07.2005
(51) Int. Cl.: C07C 229/28, C07C 227/38, C25B 3/00, A61K 31/195

(54) **METHOD OF OBTAINING CYCLIC AMINO ACIDS**
VERFAHREN ZUR GEWINNUNG CYCLISCHER AMINOSÄUREN
PROCEDE D'OBTENTION D'ACIDES AMINES CYCLIQUES

(30) Priority: 22.07.2004 ES 200401797
(43) Date of publication of application: 22.08.2007
(73) Proprietor: Menadiona , SL, 08380 Palafolls (ES)
(72) Inventor: CASTAÑE ABRADÓ, Joan Menadiona, S.L., E-08380 Palafolls (Barcelona) (ES); COSIN BORRÁS, Carlos, E-08380 Palafolls (ES)
(74) Representative: Gislon, Gabriele
(86) International application number: PCT/ES2005/000396
(87) International publication number: WO 2006/035080

(56) References cited:
- DE-B1- 1 518 068
- DE-B1- 1 518 068
- ES-T3- 2 204 533
- US-A- 3 330 749
- US-A- 4 818 409
- US-B1- 6 551 803

## Description

### Field of the Art

The present invention relates to a new industrial process for obtaining cyclic amino acids, and particularly gabapentin, with a high degree of purity.

### Prior State of the Art

Cyclic amino acids with the structure of formula (I) 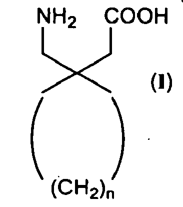 where n is an integer selected from 2, 3 and 4, have good pharmacological properties making them suitable to be used in the treatment of cerebral disorders such as epilepsy and hypokinesia, among others. They also bring about an improvement in brain functions, being particularly useful in the treatment of geriatric patients. Where n = 2 the compound has a cyclopentane ring, where n = 3, the ring is a cyclohexane ring, and where n = 4, the ring is a cycloheptane ring. In particular, gabapentin (compound of formula (I) with n = 3) is one of the compounds having the best pharmacological properties.

Compounds of formula (I), including gabapentin among them, and several processes for obtaining them are described in Spanish patent ES 443723-A. Example 1 thus describes obtaining anhydrous crystalline gabapentin from its hydrochloride by means of the treatment of an aqueous solution thereof with a basic ion exchange resin and the subsequent crystallization in an ethanol/ether mixture.

Patent documents EP 414274-A2 and EP 414275-A2 describe alternative processes for obtaining said cyclic amino acids, in which the lactam of formula (II) is used as an intermediate: 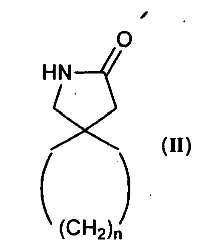 where n has the same meaning as before.

For example, the hydrolysis of lactam (II) where n = 3 with hydrochloric acid leads to obtaining gabapentin hydrochloride, which is converted into gabapentin in free amino acid form by treatment with an ion exchange resin similarly to the method anticipated in ES 443723-A.

Although other alternative processes for obtaining cyclic amino acids of formula (1) have been described (such as for example the process for preparing gabapentin described in patent document EP 432504-A1), in industrial practice processes, which have as intermediate an addition salt of the cyclic amino acid of formula (I) with a mineral acid, for example, hydrochloride, have been imposed due to their simplicity and the use of less expensive reagents.

The most significant difficulty facing the processes for preparing cyclic amino acids of formula (I) from an addition salt thereof with a mineral acid is to provide a cyclic amino acid of formula (I), and particularly gabapentin, with a quality suitable for being used as an active ingredient in pharmaceutical formulations.

As described in European patent document EP 414263-A2, the presence of a mineral acid anion (for example, chloride) may cause stability problems in cyclic amino acids of formula (I) and in their pharmaceutical formulations, due to the production of the lactam of formula (II) as an impurity.

To reduce the formation of lactam during storage, it is asserted in EP 414263-A2 that the cyclic amino acid of formula (I) must have a mineral acid anion content less than 20 ppm based on the amount of cyclic amino acid.

Patent document WO01/097612-A1 provides that pharmaceutical formulations containing gabapentin with a mineral acid anion content exceeding 20 ppm are also stable given that after one year of stability, the lactam content does not increase by more than 0.2% by weight.

Different technical solutions are found in the state of the art to eliminate the mineral acid anion and to obtain the cyclic amino acid of formula (I) in free amino acid form from an addition salt thereof with a mineral acid.

Different technical solutions are particularly found for preparing gabapentin from its addition salts with a mineral acid, for example, from hydrochloride.

Patent documents EP 340677-A2, WO 00/01660-A1, EP 1174918-A1, and the aforementioned ES 443723-A, EP 414274-A2 and EP 414275-A2 describe technical solutions based on the use of basic ion exchange resins.

In said processes, the addition salt of gabapentin with a mineral acid is dissolved in water or in a short-chain alcohol. Then said solution is eluted through the chromatographic column filled with a basic ion exchange resin. Gabapentin is finally isolated.

Patent document WO 02/34709-A2 describes the use of strongly cationic ion exchange resins. Gabapentin is thus fixed to the resin and the mineral acid anion is eliminated by elution with water.

Using this same process, patent document US2003/0119908-A1 describes stable gabapentin compositions containing less than 5 ppm of gabapentin hydrochloride and which may furthermore contain more than 20 ppm of sodium chloride, based on the amount of gabapentin.

Nevertheless, these processes for obtaining the compound have drawbacks making them rather disadvantageous at the industrial scale.

It is necessary to mention among the main drawbacks the large volumes of solvent (demineralized water or short-chain.alcohols) required, the chromatographic columns filled with ion exchange resin intended exclusively for said operation, the need to regenerate the resins, the large amount of material necessary for the metering and collection of the eluates, the evaporation of large amounts of liquids at a low temperature to prevent the formation of lactam, the long times used to reduce the mineral acid anion content to sufficiently low values, and the difficulty of being able to easily obtain gabapentin with the desired mineral acid anion content.

Patent document WO 03/089403-A1 also describes the use of ion exchange resins, but not packed in a chromatographic column, rather they are added directly as a reagent in the solid phase to a solution of the addition salt of gabapentin with a mineral acid. Then the resin is separated and a gabapentin solution in free amino acid form is obtained.

This process requires handling considerable amounts of resin, which must be regenerated before a new use, and it is difficult to obtain the end product with the desired mineral acid anion content.

Another group of technical solutions for obtaining gabapentin with a low mineral acid anion content is based on the addition of an amine to achieve selective precipitation by means of choosing the suitable solvents:
- Patent document WO 98/28255-A1 describes separating a gabapentin polymorph, called Form III, whereas the hydrochloride of the amine is maintained in dissolution.
- Patent document WO 02/44123-A1 describes that after adding the amine, gabapentin is maintained in dissolution and it is removed the precipitated amine hydrochloride.

These processes have the drawback, among others, that the obtained product may be contaminated with considerable amounts of the amine used in the neutralization step and which must then be eliminated by means of using additional purification steps.

A different technical solution for obtaining gabapentin from the addition salt thereof with a mineral acid is described in patent document WO 00/58268-A1, which proposes the diafiltration of an aqueous solution of gabapentin hydrochloride adjusted to a pH corresponding to the isoelectric point of the amino acid.

However, among the drawbacks of this process the need to work with expensive facilities using high-pressure pumps and requiring long process times so as to obtain the product within the established quality parameters can be mentioned, especially when they involve contents in the order of ppm.

Therefore, there is a need for an alternative process for obtaining cyclic amino acids substantially in non-salified form of formula (I) from its addition salts with a mineral acid, which reduces process times, handling of large volumes of solvents, and which allows obtaining said cyclic amino acids with the desired mineral acid anion content.

DE 1 518 068, US 3 330 749 and US 6 551 803 disclose electrodialysis processes for obtaining free amino acids from amino acid salts.

In particular there is a need for an alternative process for preparing gabapentin, due to its widespread commercial use.

The authors of the invention have discovered that it is possible to obtain cyclic amino acids of formula (I) with the desired mineral anion content from an addition salt of said cyclic amino acid with a mineral acid by means of an electrochemical process.

### Object of the Invention

The object of the present invention is a process for obtaining cyclic amino acids substantially in non-salified form of formula (I) by means of applying an electrochemical process.

### Description of the Invention

The process object of the invention for obtaining cyclic amino acids substantially in non-salified form of formula (I) 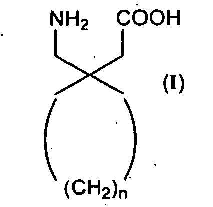 where n is an integer selected from 2, 3 and 4, is **characterized in that** it comprises:
- subjecting an aqueous solution of an addition salt of said amino acid with a mineral acid to electrolysis in an electrochemical reactor having an anodic compartment and a cathodic compartment separated by a selective anionic membrane, carrying out said electrolysis at constant voltage or constant density of the current intensity, and
- isolating the cyclic amino acid substantially in non-salified form from the aqueous solution.

In this description, the cyclic amino acid substantially in non-salified form relates to a cyclic amino acid with a mineral acid anion content not more than 100 ppm, preferably not more than 20 ppm, more preferably not more than 5 ppm.

### Starting material

The compound subjected to the electrolysis process is an addition salt of the cyclic amino acid of formula (I) with a mineral acid.

Preferably, the addition salt of the cyclic amino acid which is subjected to electrolysis is gabapentin hydrochloride and the product which is obtained is gabapentin substantially in non-salified form.

Said compounds can be prepared using any of the methods described in the state of the art, for example, in examples 1, 3 and 4 patent document ES 443723-A or in example 9 of patent document EP 414274-A2.

Throughout this description, the terms cyclic amino acid and gabapentin relate to the free amino acids.

When the amino group of the cyclic amino acid is neutralized with an acid, an addition salt of the amino acid is formed with said acid.

The addition salts of the cyclic amino acids of formula (I) can be formed with mineral acids, such as for example: hydrochloric acid, hydrobromic acid, hydroiodic acid, hydrofluoric acid, sulfuric acid, phosphoric acid; and with strong organic acids, such as for example: methanesulfonic acid, benzene sulfonic acid, p-toluenesulfonic acid.

The mineral acid forming the addition salt with the cyclic amino acid is preferably selected for the process object of the invention out of any of the hydrochloric, hydrobromic, hydroiodic, hydrofluoric acids and/or mixtures thereof. More preferably, the selected mineral acid is hydrochloric acid or hydrobromic acid.

The particularly preferred addition salts are gabapentin hydrochloride and gabapentin hydrobromide.

The solution of the addition salt of the cyclic amino acid with a mineral acid is an aqueous solution so that it is a conductor of the electric current and the electrolysis process can take place. Said aqueous solution can further comprise at least one electrolytically stable solvent miscible in water, i.e. it is stable in the electrolysis conditions. Alcohols, for example, can be mentioned among these solvents. The alcohols are preferably selected from the group formed by: methanol, ethanol, propanol, isopropanol, and/or mixtures thereof.

In the case of a hydroalcoholic solution, water is normally at least 50% of the water/alcohol mixture expressed in volume/volume. Preferably, water is the major component of the water/alcohol mixture.

The concentration of the addition salt of the cyclic amino acid with a mineral acid in the solution is determined by the solubility of said salt in the solvent selected for carrying out electrolysis.

A person skilled in the art can easily determine the most suitable concentration for carrying out the process of the invention by means of routine work.

Usually it is intended to work at the maximum concentration of the solute allowed in the chosen solvent system, and thus be able to obtain a better yield.

In the event that the starting compound is gabapentin hydrochloride, i.e. the addition salt of gabapentin with hydrochloric acid, the concentration thereof in an aqueous solution is usually comprised between 10% and 15% by weight.

### Electrochemical reactor

The electrolysis step of the process object of the invention is carried out in an electrochemical reactor having an anodic compartment and a cathodic compartment separated by a selective anionic membrane.

The process can be carried out under constant and equal pressure in both compartments during the entire electrolysis process.

The useful area of the electrochemical reactor is not a critical parameter for the process object of the invention, since the conditions of the electrochemical process can be adapted to each reactor by means of the use of techniques that are well known by a person skilled in the art. An example of a typical electrochemical reactor could be one that has a useful area comprised between 4 and 8 m².

The distance between the electrodes may be comprised between approximately 15 and 20 mm.

The anode, which is located in the in the anodic compartment, is generally formed by a metal which can resist the oxidizing conditions generated around it due to the formation of halogen molecules. The anode is preferably formed by titanium coated with noble metals such as ruthenium and iridium, which are subsequently converted into oxides by the action of the electrochemical process. These anodes are called oxygen-evolving anodes. It is also possible to use anodes of other metals that are less resistant to the oxidizing conditions present in the anode, but they would have a more limited duration and would need to be replaced sooner.

The cathode is located in the cathodic compartment, which is preferably an element with low hydrogen overvoltage, such as for example: copper, platinized titanium, stainless steel, nickel or iron. Hydrogen is generated in this compartment and accordingly the cathode does not have oxidation problems.

The selective anionic membrane is a membrane which selectively allows anions which are located in the cathodic compartment to pass through to the anodic compartment, where they are discharged in the anode, but which substantially does not allow the passage therethrough of the cyclic amino acid.

In the case of having a gabapentin hydrochloride solution in the cathodic compartment, the chloride ions move through the selective anionic membrane to the anodic compartment where they are discharged in the form of chlorine gas. The membrane substantially prevents the passage of gabapentin to the anodic compartment.

The preparation and structure of anionic membranes are thoroughly described in the book "Ion exchange membranes", T. Sata, Royal Society of Chemistry, 2004 (ISBN: 0 85404 590 2).

Commercial examples of selective anionic membranes suitable for carrying out the process of the invention are:
- IONAC MA 3475 and IONAC MA 7500, marketed by the company Sybron Chemicals.
- SYBRON MA series, marketed by the company Sybron Chemicals.
- SELEMION marketed by the company Asahi Glass Company.

### Electrochemical reactions

An aqueous, eventually hydroalcoholic, solution of the addition salt of the cyclic amino acid of formula (I) with a mineral acid, for example, gabapentin hydrochloride or hydrobromide, is introduced in the cathodic compartment of the electrochemical reactor. The anodic compartment is filled with water.

In the case of electrolysis of an aqueous solution of gabapentin hydrochloride, the electrochemical reaction occurring in the anode consists of discharging the anion chloride generating chlorine in gas form:

2 Cl⁻ → Cl₂ + 2 e⁻

In the electrolytic process, the halide anions move from the cathodic compartment through the selective anionic membrane to the anode, where they are discharged in the form of a halogen molecule. In the event that the anions of the mineral acid are chloride or bromide ions, chlorine or bromine are formed in the anode, respectively.

The presence of the halogen molecules in the anodic compartment acts against the duration of the anode and may reduce the effectiveness of the selective anionic membrane, since it may be sensitive to the oxidizing agents.

To that end, it is preferred in the process object of the invention to have a system for eliminating said halogen. A vacuum system absorbing the halogen and leading it to a trap system having a reducing solution, for example sodium bisulfite, or a sodium hydroxide solution, can be mentioned among those systems which can be used to carry out said elimination.

In the case of using gabapentin hydrochloride as a starting material in the process object of the invention, the chlorine generated in the anode can be absorbed in a sodium hydroxide solution, thereby obtaining a sodium hypochlorite solution which could be used in the synthesis of gabapentin hydrochloride according to the process described in patent document ES 443723-A by means of a Hofmann rearrangement. The same process can be applied in the case of using gabapentin hydrobromide, obtaining a sodium hypobromite solution in this case.

Another suitable system for eliminating the halogen formed in the anode comprises placing an aqueous solution of a reducing agent in the anodic compartment. In the process object of the invention, it is preferred that the reducing agent does not generate ions in the solution which may interfere with the electrochemical processes intended for preparing gabapentin. The use of a sulfur dioxide solution is especially preferred. An aqueous solution with a sulfur dioxide content comprised between 0.3% and 0.7% by weight is more preferably used.

In the cathodic compartment, the electrochemical reaction of the cathode consists of the release of hydrogen and the generation of hydroxyl ions:

2 H₂O+ 2 e⁻ → 2 OH⁻ + H₂

In the case of electrolysis of an aqueous solution of gabapentin hydrochloride, the hydroxyl ions neutralize the addition salt of gabapentin in the areas surrounding the cathode, and gabapentin (I), which is electrically neutral, is formed. The gabapentin is in equilibirum with its internal salt, which is also electrically neutral:

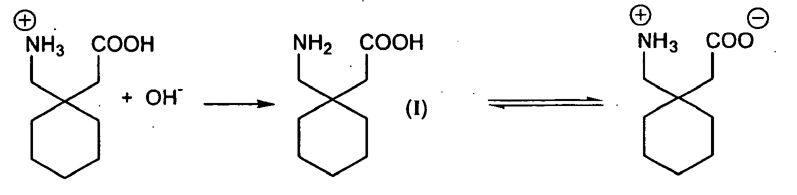

An aqueous solution of the cyclic amino acid is thus obtained in the cathodic compartment, said amino acid being able to be isolated from such solution by using techniques well known by a person skilled in the art, such as for example the evaporation under a vacuum and at a temperature of not more than 50° C.

### Parameters of the electrolytic process

Electrolytic processes may generally take place at a constant voltage or at a constant density of the current intensity.

The voltage or potential difference between the two electrodes is what generates a movement of ions and therefore a current intensity.

The density of the current intensity is the intensity of the current in relation to the surface unit of the electrode. In the case of an electrolytic reaction, the current intensity is provided by the ions which are dissolved in the solution.

A person skilled in the art may establish the working conditions in a routine manner so that the process is economically viable and so that oxidation of the cyclic amino acid in the anode is prevented.

In the event of carrying out the electrolytic process with a constant density of the current intensity, the electrochemical reactor and the parameters of the density of the intensity and the voltage are adapted so as to reduce the discharge of the cyclic amino acid in the anode, for example, not more than 40 V, and to have a process that is economically advantageous, for example, with a voltage of not less than 3 V.

The electrolytic process preferably occurs at a constant voltage in the process object of the invention. The voltage used is preferably comprised between 3 and 20 V. It is more preferably comprised between 8 and 13 V.

Although the use of voltages of less than 3 V in the electrolytic process has no repercussions in terms of the quality characteristics of the cyclic amino acid obtained, it does require larger industrial facilities or longer process times.

Carrying out the electrolytic process at higher voltages, for example in the order of 40 V, contributes to a yield loss because it increases the tendency of the cyclic amino acid to move to the anode and oxidize.

Taking into consideration that the voltage is equal to the product of intensity times resistance, a constant voltage with the process achieves that the intensity of the system varies according to the resistance offered by the solution contained in the cathodic compartment.

As the halide ions move to the anodic compartment, resistance is increased, or in other words, the conductivity of the solution of the addition salt of the cyclic amino acid with a mineral acid located in the cathodic compartment decreases. The conductivity of the solution is directly proportional to the concentration of the halide ions therein. The initial conductivity values are typically comprised between 40 and 60 mS. However at the end of the process they are usually comprised between 150 and 300 µS.

Accordingly, the initial density of the current intensity is set by the voltage and the concentration of the addition salt of the cyclic amino acid with a mineral acid in the cathodic compartment. The typical values for the density of the current intensity at the start are comprised between 500 A/m² and 2,000 A/m², preferably between 1,000 A/m² and 1,500 A/m².

Throughout the electrolytic process, the density of the current intensity is gradually reduced as the halide ions move towards the anodic compartment, and the cathodic compartment becomes enriched in cyclic amino acid which has no electric charge, though it is in equilibrium with its internal salt, which also has no net electric charge. The typical values for the density of the current intensity at the end of the process are comprised between 30 A/m² and 70 A/m².

The electrochemical reaction ends because the cyclic amino acid solution is not conductive and, as there is no conductivity, the density of the current intensity reaches the aforementioned minimum values.

The pH in the cathodic compartment is simultaneously modified as the electrolytic process progresses. The initial pH is approximately 1 because it contains a solution of an addition salt of the cyclic amino acid with a mineral acid, and the pH at the end of the electrolytic process is close to the neutral area because it contains a solution of the cyclic amino acid.

The progressive reduction of the conductivity and the increase in the pH value can be observed in the cathodic compartment during the entire process object of the invention.

As previously mentioned, conductivity is directly related to the concentration of anions of the mineral acid in the cathodic compartment, such that it is possible to control it continuously, being able to end the reaction at the same moment in which the concentration of anions in the solution corresponds with the concentration established as a quality parameter. Said concentration may be more than or less than 20 ppm of mineral acid anion with respect to the cyclic amino acid, as deemed appropriate by a person skilled in the art, as indicated in the section of this description which corresponds to the state of the art.

In the process object of the invention, the electrolytic process takes place at a temperature at which the formation of lactam as an impurity is substantially prevented. Said temperature is preferably not more than 50° C. It is more preferably comprised between 10° C and 25° C.

The temperature can be maintained between the established values by means of using apparatus that are well known by a person skilled in the art. Plate heat exchangers, which cool the solution through an external circuit, can be mentioned among such apparatus.

The selected temperature interval also minimizes the passage of the addition salt of the cyclic amino acid with a mineral acid through the selective anionic membrane, therefore the process yield is better.

### Isolation of the cyclic amino acid substantially in non-salified form

In the process object of the invention, the cyclic amino acid substantially in non-salified form present in the aqueous, eventually hydroalcoholic, solution of the cathodic compartment is isolated by means of techniques that are well known by a person skilled in the art, such as evaporation by distillation, atomization or turbo-drying.

For example, in the case of evaporation by distillation the aqueous solution of the cyclic amino acid is evaporated to dryness by means of applying a vacuum so as to prevent temperatures of more than 50° C, which could generate lactam as an impurity. The aqueous solution is typically evaporated at a temperature comprised between 10° C and 25° C. The applied vacuum is generally comprised between 133 and 1,333 Pa (1 - 10 mmHg).

The cyclic amino acid substantially in non-salified form can then be crystallized in a solvent selected from the group formed by methanol, ethanol or isopropanol.

The cyclic amino acid substantially in non-salified form obtained with the process object of the invention has typical mineral acid anion contents related to the amount of cyclic amino acid, not more than 10 ppm, values of not more than 3 ppm being usual. In terms of the lactam content, typical values are under 0.01% by weight, being able to reach values of not more than 0.001%.

The process object of the invention allows preparing gabapentin substantially in non-salified form with a mineral acid anion content of not more than 20 ppm related to the amount of gabapentin. The mineral acid anion content is preferably not more than 10 ppm. A mineral acid anion content close to 0 ppm can even be obtained, i.e. the complete elimination of the anion, which would be difficult in the case of using ion exchange resins, can be attained.

The cyclic amino acid losses during the process are generally comprised below 2%.

The process object of the invention allows obtaining cyclic amino acids substantially in non-salified form of formula (I) by means of a simple process, with a good yield and high quality; with very low lactam contents and with a mineral acid anion content which is in accordance with the quality parameters established by the skilled person, being able to reach not more than 3 ppm. This ensures excellent stability both of the product and of its pharmaceutical formulations.

With the process object of the invention, the mineral acid anion content can be controlled on line and the reaction can be stopped when the pre-established parameters have been reached.

The following example is set forth for the purpose of providing a person skilled in the art with a detailed explanation of a specific embodiment within the invention and of the benefits obtained with it.

### Example

### Example 1 Preparation of gabapentin

An aqueous solution comprising 200 kg of gabapentin hydrochloride and 1,500 kg of deionized water is loaded in the cathodic compartment, and 5,000 kg of an aqueous solution of sulfur dioxide at 0.5% by weight is loaded in the anodic compartment in an electrochemical reactor with a useful area of 6 m² equipped with an anodic compartment having oxygen-evolving anodes and with a cathodic compartment with stainless steel cathodes, separated by a selective anionic membrane SYBRON MA series (marketed by Sybron Chemicals).

The circulation pumps are started up and a constant voltage of 11 V is set for the electrolysis.

The values of the following parameters are recorded during the electrochemical process: density of the current intensity, conductivity and pH of the cathodic compartment.

The density of the intensity gradually decreases from the initial value of 1,250 A/m² to a final value of 50 A/m². The conductivity of the cathodic compartment is simultaneously reduced from an initial value of 50 mS to a final value of approximately 200 µS, and the pH value goes from 1 to approximately 7.

The electrolysis is carried out maintaining the temperature of the solution of the cathodic compartment at approximately 15° C by means of an external plate heat exchanger.

Once the electrolysis has ended with the described parameters, the solution of the cathodic compartment is discharged to an evaporator.

The water is evaporated to dryness using a vacuum comprised between 133 and 1,333 Pa (1 - 10 mmHg) and a temperature comprised between 10° C and 25° C.

Once the evaporation has ended, 1,500 kg of isopropanol are added and it is heated as little time as possible at the reflux temperature to check that solubility is total. Then the solution is cooled at 0° C and gabapentin is obtained by precipitation.

The obtained solid is filtered, washed with anhydrous isopropanol and dried in a rotary drum with a high vacuum at room temperature.

Approximately 150 kg of gabapentin are obtained.

The chloride content is determined by titration according to the Mohr method and a value of less than 10 ppm is obtained.

The lactam content is determined by HPLC and a value of less than 0.01% is obtained.

## Claims

1. A process for obtaining cyclic amino acids substantially in non-salified form of formula (I) 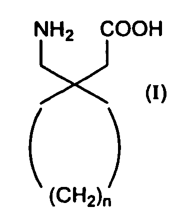 wherein n is an integer selected from 2, 3 and 4,
**characterized in that** it comprises:
- subjecting an aqueous solution of an addition salt of said amino acid with a mineral acid to electrolysis in an electrochemical reactor having an anodic compartment and a cathodic compartment separated by a selective anionic membrane, carrying out said electrolysis at constant voltage or at a constant density of the current intensity, and
- isolating the cyclic amino acid substantially in non-salified form from the aqueous solution.

2. A process according to claim 1, **characterized in that** the electrolysis takes place at a constant voltage.

3. A process according to claim 2, **characterized in that** the voltage used is comprised between 3 and 20 V.

4. A process according to claim 3, **characterized in that** the voltage used is comprised between 8 and 13 V.

5. A process according to claims 1 to 4, **characterized in that** the electrolysis is carried out at a temperature that is not more than 50° C.

6. A process according to claim 5, **characterized in that** the temperature is comprised between 10° C and 25° C.

7. A process according to claims 1 to 6, **characterized in that** the anodic compartment comprises an aqueous solution of a reducing agent.

8. A process according to claim 7, **characterized in that** the reducing agent is sulfur dioxide.

9. A process according to claims 1 to 8, **characterized in that** the mineral acid forming the addition salt with the cyclic amino acid is selected out of any of the hydrochloric, hydrobromic, hydroiodic, hydrofluoric acids and/or mixtures thereof

10. A process according to claim 9, **characterized in that** the selected mineral acid is hydrochloric acid or hydrobromic acid.

11. A process according to claims 1 to 10, **characterized in that** the aqueous solution further comprises at least one electrolytically stable solvent miscible with water.

12. A process according to claim 11, **characterized in that** the solvent miscible with water is an alcohol selected from the group formed by: methanol, ethanol, propanol, isopropanol and/or mixtures thereof.

13. A process according to claim 12, **characterized in that** water is at least 50% by volume of the water/alcohol mixture.

14. A process according to claims 1 to 13, **characterized in that** the addition salt of cyclic amino acid which is subjected to electrolysis is gabapentin hydrochloride and the product which is obtained is gabapentin substantially in non-salified form.

15. A process according to claim 14, **characterized in that** the concentration of gabapentin hydrochloride in the aqueous solution is comprised between the 10% and 15% by weight.

16. A process according to claim 1, **characterized in that** the addition salt of cyclic amino acid which is subjected to electrolysis is gabapentin hydrochloride, the voltage used is comprised between 3 and 20 V, and the temperature is not more than 50° C.

17. A process according to claim 16, **characterized in that** the voltage used is comprised between 8 and 13 V, and that the temperature is comprised between 10° C and 25° C.

## Patentansprüche

1. Verfahren zum Erhalten von zyklischen Aminosäuren im Wesentlichen in nicht salzbildender Form der Formel (I) 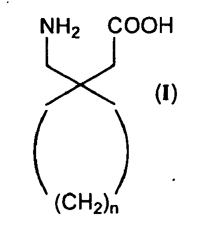 in welcher n eine aus 2, 3 und 4 ausgewählte ganze Zahl ist,
**dadurch gekennzeichnet, dass** es Folgendes umfasst:
- Unterziehen einer wässrigen Lösung eines Additionssalzes der genannten Aminosäure mit einer Mineralsäure einer Elektrolyse in einem elektrochemischen Reaktor, der einen Anodenraum und einen Kathodenraum hat, die durch eine wahlweise anionische Membran getrennt sind, wobei die genannte Elektrolyse bei konstanter Spannung oder bei konstanter Dichte der Stromstärke durchgeführt wird, und
- Isolieren der zyklischen Aminosäure, im Wesentlichen in nicht salzbildender Form, von der wässrigen Lösung.

2. Verfahren nach Anspruch 1, **dadurch gekennzeichnet, dass** die Elektrolyse bei einer konstanten Spannung stattfindet.

3. Verfahren nach Anspruch 2, **dadurch gekennzeichnet, dass** die verwendete Spannung zwischen 3 und 20 V beträgt.

4. Verfahren nach Anspruch 3, **dadurch gekennzeichnet, dass** die verwendete Spannung zwischen 8 und 13 V beträgt.

5. Verfahren nach den Ansprüchen 1 bis 4, **dadurch gekennzeichnet, dass** die Elektrolyse bei einer Temperatur von nicht mehr als 50 °C durchgeführt wird.

6. Verfahren nach Anspruch 5, **dadurch gekennzeichnet, dass** die Temperatur zwischen 10 °C und 25 °C beträgt.

7. Verfahren nach den Ansprüchen 1 bis 6, **dadurch gekennzeichnet, dass** der Anodenraum eine wässrige Lösung eines Reduktionsmittels umfasst.

8. Verfahren nach Anspruch 7, **dadurch gekennzeichnet, dass** das Reduktionsmittel Schwefeldioxid ist.

9. Verfahren nach den Ansprüchen 1 bis 8, **dadurch gekennzeichnet, dass** die Mineralsäure, welche das Additionssalz mit der zyklischen Aminosäure bildet, aus der Gruppe bestehend aus Chlorwasserstoff-, Bromwasserstoff-, Jodwasserstoff- und Fluorwasserstoffsäuren und/oder deren Mischungen ausgewählt wird.

10. Verfahren nach Anspruch 9, **dadurch gekennzeichnet, dass** die ausgewählte Mineralsäure Chlorwasserstoffsäure oder Bromwasserstoffsäure ist.

11. Verfahren nach den Ansprüchen 1 bis 10, **dadurch gekennzeichnet, dass** die wässrige Lösung weiterhin zumindest ein elektrolytisch stabiles Lösungsmittel umfasst, das wassermischbar ist.

12. Verfahren nach Anspruch 11, **dadurch gekennzeichnet, dass** das wassermischbare Lösungsmittel ein Alkohol ist, das aus folgender Gruppe ausgewählt ist: Methanol, Ethanol, Propanol, Isopropanol und/oder deren Mischungen.

13. Verfahren nach Anspruch 12, **dadurch gekennzeichnet, dass** der Wasseranteil in der Wasser-Alkohol-Mischung zumindest 50 Volumenprozent einnimmt.

14. Verfahren nach den Ansprüchen 1 bis 13, **dadurch gekennzeichnet, dass** das Additionssalz der zyklischen Aminosäure, welches einer Elektrolyse unterzogen wird, Gabapentin-Hydrochlorid, und das erhaltene Produkt Gabapentin im Wesentlichen in nicht salzbildender Form ist.

15. Verfahren nach Anspruch 14, **dadurch gekennzeichnet, dass** die Konzentration an Gabapentin-Hydrochlorid in der wässrigen Lösung zwischen 10 und 15 Gewichtsprozent beträgt.

16. Verfahren nach Anspruch 1, **dadurch gekennzeichnet, dass** das Additionssalz der zyklischen Aminosäure, welches der Elektrolyse unterzogen wird, Gabapentin-Hydrochlorid ist, die verwendete Spannung zwischen 3 und 20 V beträgt, und die Temperatur nicht mehr als 50 °C beträgt.

17. Verfahren nach Anspruch 16, **dadurch gekennzeichnet, dass** die verwendete Spannung zwischen 8 und 13 V beträgt, und dass die Temperatur zwischen 10 °C und 25 °C beträgt.

## Revendications

1. Une méthode pour obtenir des acides aminocycliques sous forme sensiblement non salifiée à formule (I) 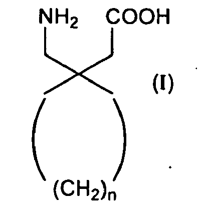 où n est un nombre entier choisi parmi 2, 3 et 4,
**caractérisée en ce qu'**elle comporte
- soumettre une solution aqueuse d'un sel additif de cet aminoacide avec un acide minéral à l'électrolyse dans un réacteur électrochimique ayant un compartiment anodique et un compartiment cathodique séparés par une membrane anionique sélective, effectuant cette électrolyse à une tension constante ou à une densité constante d'intensité de courant, et
- isoler l'acide aminocyclique sous forme sensiblement non salifiée à partir d'une solution aqueuse.

2. Une méthode conformément à la revendication 1, **caractérisée en ce que** l'électrolyse a lieu à une tension constante.

3. Une méthode conformément à la revendication 2, **caractérisée en ce que** la tension utilisée est comprise entre 3 et 20 V.

4. Une méthode conformément à la revendication 3, **caractérisée en ce que** la tension utilisée est comprise entre 8 et 13 V.

5. Une méthode conformément aux revendications 1 à 4, **caractérisée en ce que** l'électrolyse est effectuée à une température non supérieure à 50° C.

6. Une méthode conformément à la revendication 5, **caractérisée en ce que** la température est comprise entre 10° C et 25° C.

7. Une méthode conformément aux revendications 1 à 6, **caractérisée en ce que** le compartiment anodique comporte une solution aqueuse d'un agent réducteur.

8. Une méthode conformément à la revendication 7 **caractérisée en ce que** l'agent réducteur est du dioxyde de sulfure.

9. Une méthode conformément aux revendications 1 à 8, **caractérisée en ce que** l'acide minéral formant le sel additif avec l'acide aminocyclique est choisi d'un quelconque des acides hydrochlorique, hydrobromique, hydroiodique, hydrofluorique et/ou leurs mélanges.

10. Une méthode conformément à la revendication 9, **caractérisée en ce que** l'acide minéral choisi est l'acide hydrochlorique ou l'acide hydrobromique.

11. Une méthode conformément aux revendications 1 à 10, **caractérisée en ce que** la solution aqueuse comporte en plus au moins un solvant électrolitiquement stable miscible avec de l'eau.

12. Une méthode conformément à la revendication 11, **caractérisée en ce que** le solvant miscible avec de l'eau dans un alcool est choisi du groupe formé par : le méthanol, éthanol, propanol, isopropanol et/ou leurs mélanges.

13. Une méthode conformément à la revendication 12, **caractérisée en ce que** l'eau est au moins 50% par volume du mélange d'eau/alcool.

14. Une méthode conformément aux revendications 1 à 13, **caractérisée en ce que** le sel additif de l'acide aminocyclique qui est soumis à l'électrolyse est l'hydrochlorure de gabapentine et le produit qui est obtenu est la gabapentine sous la forme sensiblement non salifiée.

15. Une méthode conformément à la revendication 14, **caractérisée en ce que** la concentration d'hydrochlorure de gabapentine dans la solution aqueuse est comprise entre les 10% et 15% par poids.

16. Une méthode conformément à la revendication 1, **caractérisée en ce que** le sel additif de l'acide aminocyclique qui est soumis à l'électrolyse est l'hydrochlorure de gabapentine, la tension utilisée est comprise entre 3 et 20 V et la température n'est pas supérieure à 50°C.

17. Une méthode conformément à la revendication 16 **caractérisée en ce que** le voltage utilisé est compris entre les 8 et 13 V et que la température est comprise entre les 10°C et 25°C.
